# EUROPÄISCHE PATENTANMELDUNG

(11) **EP 1 241 226 A1**
(43) Veröffentlichungstag der Anmeldung: **18.09.2002**
(21) Anmeldenummer: 01106620.6
(22) Anmeldetag: 16.03.2001
(51) Int. Cl.: C08L 71/02, C08L 89/06

(54) **Verwendung von Polyol als Gelbildner, Zusammensetzung für die Herstellung von Filmen und Formkörpern, Verfahren zum Herstellen eines kaltwasserlöslichen Gelatinegels und Verfahren zum Herstellen einer Kapsel**

(71) Anmelder: Swiss Caps Rechte und Lizenzen AG, 9533 Kirchberg (CH)
(72) Erfinder: Maier, Hans-Jürgen Dr., 8636 Wald-Oberholz (CH)
(74) Vertreter: Wenger, René

(57) **Zusammenfassung**

Die vorliegende Erfindung betrifft die Verwendung von Polyethylenglykol mit Molekulargewichten im Bereich von 200 bis 35000 g/mol (Gewichtsmittel) als Gelbildner für insbesondere native Gelatine oder Gelatinehydrolysate. Die Gelatinehydrolysate haben Bloom-Zahlen im Bereich zwischen 40 und 120, bevorzugt um die 100. Die gelbildende Zusammensetzung ist, insbesondere in der Ausführungsform mit Gelatinedepolymerisaten besonders geeignet für die Herstellung von Formkörpern, insbesondere Kapseln mit einteiliger Kapselhülle, welche bei Temperaturen unterhalb von 40°C in Wasser löslich sind und dabei ihren Inhaltsstoff freisetzen.

## Beschreibung

Gegenstand der vorliegenden Erfindung ist die Verwendung von wenigstens einem Polyol als Gelbildner, eine Zusammensetzung für die Herstellung von Filmen und Formkörpern, sowie Verfahren zum Herstellen eines kaltwasserlöslichen Gelatinegels und Verfahren zum Herstellen einer Kapsel.

Verpackungsmittel und Formkörper aus den unterschiedlichsten Biopolymeren (Polymere natürlichen Ursprungs) sind heute als Einmalverpackungen und Dosierungsmittel im Einsatz. Die Anwendungen sind vielfältig und umfassen die Gebiete Gesundheit, Nahrung und Technik.

Das Polypeptid Gelatine ist aufgrund seiner nahezu idealen Eigenschaften in Bezug auf Verarbeitbarkeit, Stabilität, Kompatibilität mit einer Vielzahl von Substanzen sowie seiner Verträglichkeit für menschliche und tierische Organismen in Form von Weich- und Hartkapseln weitverbreitet im Einsatz.

Wässrige, insbesondere native Gelatine-Lösungen erstarren bereits bei einer Konzentration von ca. 1 Gew.% Gelatine (bezogen auf das Gesamtgewicht der Mischung) bei Temperaturen unterhalb von 35°C zu einer gallertartigen Masse (Gel), die erst wieder bei Überschreiten dieser Temperatur in einen flüssigen Zustand überführt werden kann (Sol). Unterhalb dieses, in der vorliegenden Anmeldung als Gelpunkt bezeichneten Umwandlungspunktes, ist lediglich ein Quellvorgang zu beobachten.

Die mangelnde Wasserlöslichkeit von auf Gelatine basierenden Filmen bzw. allgemein Formkörpern unterhalb einer Temperatur von 40°C, kann besonders für technische Anwendungen nachteilig sein. So sind beispielsweise sogenannte Waschkugeln im Einsatz, in denen eine für einen Waschgang abgemessene Menge an Flüssigwaschmittel in Gelatine-Hüllen eingeschlossen ist. Die temperaturabhängige Wasserlöslichkeit von Gelatine spielt bei Waschtemperaturen von 60°C bis 95°C keine Rolle. Jedoch ist das Freisetzen des Waschmittels bei Fein- und Wollwaschgängen, die unterhalb einer Wassertemperatur von 40°C und oftmals mit verkürzten Waschzeiten (im Gegensatz zu Vollwaschgängen) durchgeführt werden, verhindert.

Aufgabe der vorliegenden Erfindung ist es, die Nachteile des Bekannten zu vermeiden.

Insbesondere ist es Aufgabe der Erfindung, Zusammensetzungen auf Basis von Biopolymeren bereitzustellen, die sich zur Herstellung von Formkörpern beliebiger Art eignen.

Eine weitere Aufgabe besteht darin, Zusammensetzungen auf Basis von Biopolymeren und daraus hergestellte Formkörper bereitzustellen, deren Lösetemperatur in Wasser einstellbar ist und die sich insbesondere in Wasser bei Temperaturen kleiner oder gleich 40°C lösen oder sich insbesondere bei hohen Temperaturen wie z.B. im kochenden Wasser nicht auflösen. Die Temperaturabhängigkeit des Gelpunktes der Zusammensetzung soll damit einstellbar sein.

Eine weitere Aufgabe besteht darin, Zusammensetzungen auf Basis von Biopolymeren bereitzustellen, die mittels konventioneller Filmerzeugungs- bzw. Formungs- und Verkapselungseinrichtungen zu Formkörpern verarbeitbar sind. Eine weitere Aufgabe besteht in der Bereitstellung solcher Zusammensetzungen, die im Rotary-Die-Verfahren zu Weichkapseln formbar sind.

Insbesondere sollen diese Zusammensetzungen auf Basis von Biopolymeren bei Raumtemperatur filmbildend sein. Eine weitere Aufgabe besteht darin, dass die filmbildenden Zusammensetzungen auf Basis von Gelatine und ihren Derivaten herstellbar sind.

Eine weitere Aufgabe besteht darin, ein Verfahren zur Herstellung einer Zusammensetzung bereitzustellen, welche sich zur Herstellung von Formkörpern beliebiger Art eignet und deren Lösetemperatur in Wasser einstellbar ist.

Diese Aufgaben werden durch die Merkmale der unabhängigen Patentansprüche gelöst.

Insbesondere werden sie gelöst durch die Verwendung mindestens eines Polyols als Gelbildner für flüssige Mischungen enthaltend mindestes ein aminogruppenhaltiges Polymer.

In einer bevorzugten Ausführungsform umfasst das Polyol Polyethylenglykol mit einem Molekulargewicht im Bereich von 200 bis 35000g/mol (Gewichtsmittel) bevorzugt von 400 bis 20000 g/mol und noch bevorzugter von 800 bis 2000 g/mol.

Als Polyole können abgesehen von Polyethylenglykol auch Polypropylenglykol oder Mischungen dieser beiden Polymere verwendet werden.

Die Gelbildung erfolgt unabhängig von der chemischen Struktur der Polymeren, solange das Polymer Aminogruppen trägt, wie z.B.

Chitosan. In einer bevorzugten Ausführungsform wird das Polymer jedoch ausgewählt aus der Gruppe der Polypeptide.

Insbesondere ist das Polypeptid ausgewählt aus der Gruppe bestehend aus nativer Gelatine und Gelatinehydrolysat.

Im Zuge der vorliegenden Erfindung gelten folgende Definitionen:

Der Begriff Mischung soll alle Bestandteile abgesehen vom Polyol umfassen. Mit der Bezeichnung Zusammensetzung ist die Mischung inklusive Polyol, insbesondere Polyethylenglykol, gemeint.

Die Bloom-Zahl ist ein Mass für die Gelbildungstendenz der Gelatine (Standard methods for sampling and testing gelatines, Herausgeber: Gelatine Manufacturers Institute of America Corporation, New York, 1977).

Unter dem Begriff Gel sollen formbeständige, leicht deformierbare disperse Systeme verstanden werden, die mindestens zwei Komponenten, die dispergierte Substanz und das Dispersionsmittel enthalten. In Gelen ist, im Gegensatz zu den Solen, die dispergierte Substanz nicht mehr frei im Dispersionsmittel beweglich; sie bildet in diesem Zustand keine Partikel im Sinne selbständiger kinetischer Einheiten aus, sondern ist in Folge besonderer struktureller Anordnung, wie z.B. Ausbildung eines dreidimensionalen Netzwerkes, räumlich fixiert. Durch derartige Strukturen ist das Dispersionsmittel immobilisiert, und es entsteht ein Objekt, das formbeständig ist, obwohl es zum weitaus überwiegenden Teil aus Flüssigkeit besteht.

Der Begriff Sol bezeichnet die zum Gel korrespondierende Phase im flüssigem Zustand, die unter vorbestimmten Bedingungen in ein Gel überführbar ist.

Somit werden als Gelbildner solche Mittel bezeichnet, die in der Lage sind Substanzen, in Gele zu überführen.

Unter dem Begriff Gelpunkt soll der Temperaturbereich bzw. der Temperaturpunkt bezeichnet werden, bei dem der Übergang vom Sol zum Gel bzw., wenn reversibel, vom Gel zum Sol stattfindet.

Der Begriff Vernetzung bezeichnet die Ausbildung kovalenter und nicht kovalenter (koordinativer, ionischer, physikalischer, salzartiger) Bindungen.

Der Begriff Film bezeichnet eine zusammenhängende Beschichtung, die durch Auftrag mindestens einer Schicht auf einem Untergrund entsteht.

Der Begriff Gelatinehydrolysat steht für depolymerisierte Gelatine, auch wenn die Verringerung des Polymerisationsgrades durch andere Methoden als durch Hydrolyse erfolgt.

Schon native Gelatine kann - abhängig von der Kollagenquelle und Herstellungsweise - eine Bandbreite an Polymerisationsgraden besitzen. Als Gelatinehydrolysat im Sinne der vorliegenden Erfindung wird Gelatine jeglicher Kettenlänge verstanden, wenn sie zur Herabsetzung der Lösetemperatur einer Depolymerisierung unterzogen worden ist.

Der Begriff "Gelatine" soll als Oberbegriff für die Gruppe Gelatinehydrolysat und native Gelatine stehen.

Aminogruppenhaltige Polymere/Polymerhydrolysate bezeichnen Polymere natürlichen oder synthetischen Ursprungs, welche wenigstens eine primäre, sekundäre oder tertiäre Aminogruppe kovalent am Polymergerüst gebunden haben.

Als Polymer bzw. mit dem Präfix "Poly-" werden auch Polymerisationsgrade umfasst, die anderweitig bereits als Oligomere bezeichnet werden, also relativ kurzkettige Polymere sind.

Unter Lösen soll die Fähigkeit der Zusammensetzung verstanden werden, vorhandene Inhaltsstoffe freizusetzen, wenn das Gel als Formkörperhülle verarbeitet wird.

Ohne auf dieses Modell festgelegt zu werden, wird angenommen, dass Wasserstoffbrückenbindungen zwischen den Sauerstoffen des Polyols und den Aminogruppierungen der nativen bzw. depolymerisierten Polymere ausgebildet werden, wodurch ein Netzwerk geschaffen wird. Nichtkovalente Bindungen sorgen für zusätzliche Vernetzungsstellen zwischen den depolymerisierten Polymeren und Polyol bzw. zwischen depolymerisierten Polymeren.

Überraschenderweise hat sich gezeigt, dass die Zugabe von Polyolen und insbesondere von Polyethylenglykol zu Mischungen, die "Gelatine" enthalten einen Übergang der vor Zugabe flüssigen Mischungen in den Gelzustand bewirkt, ohne dass die Temperatur der Mischung geändert werden muss. Das bedeutet, dass bei der minimalen Temperatur, bei der die Mischung für das Auge des Betrachters ohne unterscheidbare Phasen (im Sinne der Erfindung, homogen) vorliegt, die Mischung durch Zugabe von Polyolen in den Gelzustand überführt werden kann.

Das so entstandene Gel kann bei Erhöhung der Temperatur jedoch in den Solzustand übergehen.

In manchen Fällen erzeugt die Zugabe von Polyolen, insbesondere Polyethylenglykol, auch dann noch eine Gelbildung, wenn die minimale Temperatur wesentlich überschritten ist.

In einer Ausführungsform kann die Mischung im wesentlichen aus Gelatine und/oder Gelatinehydrolysat bestehen. Einzig können noch die Wasseranteile vorhanden sein, die ohne vorgängige Trocknung der Gelatine bzw. dem Hydrolysat vorhanden sind. Die der Gelatine eigenen "inhärenten" Wasseranteile betragen in der Regel zwischen 4 und 7 Gew.% bezogen auf das Gewicht der Gelatine.

Die Mischung kann aber auch in einer weiteren Ausführungsform grössere Mengen Wasser enthalten, ohne dass die Gelbildung bei Zugabe von Polyolen, insbesondere Polyethylenglykol beeinträchtigt wird. Die Mischung kann zwischen 2 und 80 Gew.% Wasser bezogen auf das Gesamtgewicht der Mischung beinhalten, bevorzugter beträgt der Wasseranteil 5 bis 50 Gew.% und noch bevorzugter 10 bis 20 Gew.%.

Die Gelbildung der "Gelatine" findet unter Einfluss des Polyethylenglykols unabhängig vom Polymerisationsgrad der Gelatine statt. Der Polymerisationsgrad ist jedoch mitbestimmend für die minimale Temperatur, bei der das Polymer/Polymerhydrolysat flüssig vorliegt.

Je geringer die Kettenlänge, d.h. je geringer der Polymerisationsgrad der jeweiligen "Gelatine", desto tiefer ist die Temperatur bei der die "Gelatine" flüssig vorliegt.

Die Kettenlänge spiegelt sich ausserdem in den Eigenschaften des nach Polyolzugabe, insbesondere Polyethylenzugabe, erhaltenen Gels. Sie ist ein Mass dafür, bei welcher Temperatur das entstandene Gel wasserlöslich ist. Je tiefer der Polymerisationsgrad der "Gelatine", desto niedriger die Lösetemperatur des Gels.

Ein ähnlicher Zusammenhang besteht in bezug auf die Bloom-Zahl der "Gelatine". Je tiefer die Bloom Zahl, desto tiefer ist die Temperatur bei der die "gelatine" wasserlöslich ist bzw. flüssig vorliegt, desto weniger ist aber auch die Gelbildungstendenz.

"Gelatine" hoher Bloom-Zahl erreicht den flüssigen Zustand unabhängig von der vorhandenen Menge Wasser erst bei erhöhten Temperaturen. Die Zugabe von Polyethylenglykol erfolgt dann ebenfalls bei erhöhten Temperaturen.

Die Zugabe von Polyethylenglykol eines mittleren Molekulargewichts im Bereich von 200 g/mol bis 35000 g/mol (Gewichtsmittel) zu einer Mischung enthaltend Gelatinehydrolysat einer Bloom-Zahl im Bereich von 40 bis 120, bevorzugt 100 führt zu Gelen, die bei Raumtemperatur wasserlöslich sind. Bevorzugt hat das Polyethylenglykol ein mittleres Molekulargewicht von 400 g/mol bis 20000 g/mol und noch bevorzugter 800 g/mol bis 2000 g/mol. Am besten geeignet ist ein mittleres Molekulargewicht von 800 g/mol.

Die Zugabe von Polyehtylenglykol mit einem mittleren Molekulargewicht im Bereich von 200 bis 35000 g/mol, zu einer Mischung enthaltend "Gelatine" einer Bloom-Zahl von mindestens 170 bevorzugter 200 führt in einer weiteren Ausführungsform zu Gelen, die bei Raumtemperatur nicht mehr im Wasser löslich sind. Je höher die Bloom-Zahl der Gelatine, desto höher die Temperatur in der der Übergang zum Gel stattfindet. Das mittlere Molekulargewicht des Polyethylenglykols ist bevorzugt im Bereich von 400 bis 20000 g/mol und noch bevorzugter im Bereich von 800 bis 2000 g/mol.

Bloom-Zahlen von grösser oder gleich 200 führen zu Gelen, die selbst in kochendem Wasser (95°C) nicht mehr löslich sind.

"Gelatine" einer Bloom-Zahl von Null führt bei Zugabe von Polyethylenglykol (Mw = 800 g/mol) ebenfalls zu Gelen, die aber z.B. bei Raumtemperatur nicht mehr zu selbsttragenden Filmen verarbeitbar sind.

Die Art der Depolymerisierung der Amingruppen enthaltenden Polymeren, insbesondere aus Gelatine, spielt für die vorliegende Erfindung keine Rolle. Die Hydrolyse kann säure-, enzymatisch-, und/oder basenkatalysiert und/oder mittels thermisch und/oder mechanischer Degradation des Polymeren ablaufen. Die Depolymerisation kann unter Ausnützung des im Polymeren vorhanden Wasseranteils, der abhängig vom Polymeren und von den Lagerbedingungen in der Regel zwischen 4% und 15% beträgt, durchgeführt werden. Alternativ kann die Depolymerisation aber auch unter Zugabe von Wasser in wässriger Suspension ablaufen. Die Depolymerisation sollte jedoch nicht unter solchen Bedingungen durchgeführt werden, dass es zur Abspaltung der Amingruppen vom Polymergerüst kommt.

In einer bevorzugten Ausführungsform wird hochbloomige Gelatine, insbesondere einer Bloom-Zahl von mindestens 170, während mehrerer Tage, vorzugsweise unter gleichmässigem Rühren, bei ca. 100°C gehalten. Eine Wasserzugabe erfolgt nicht, die Depolymerisation verläuft mit dem in der eingesetzten Gelatine vorhandenen Wasseranteil. Eine Probenentnahme erfolgt in zeitlich gleichmässigen Abständen. Der Fortgang der Depolymerisierung wird dabei durch Löslichkeitstests in 15° - 20°C warmen Wasser (Leitungswasser) überprüft. Löst sich das Hydrolysat bei dieser Temperatur im Wasser, wird die Depolymerisierung abgebrochen. Dies ist bei hochbloomiger Gelatine bei 100°C nach ca. 7 Tagen der Fall.

Wird des Hydrolysat bei 20°C unter Zugabe von Polyethylenglykol in ein Gel überführt, zeigt dieses Gel "Kaltwasserlöslichkeit", d.h. zeigt Löslichkeit unter 25°C, bevorzugt bei 20°C.

Teil der vorliegenden Erfindung ist weiterhin eine bei einer vorbestimmbaren Temperatur gel- und filmbildende Zusammensetzung für die Herstellung von Formkörpern, welche mindestens ein Polyol, insbesondere Polyethylenglykol mit einem Molekulargewicht im Bereich von 200 bis 35000 g/mol (Gewichtsmittel) enthält und mindestens ein Polypeptid ausgewählt aus der Gruppe umfassend native Gelatine und Gelatinehydrolysat.

In einer bevorzugten Ausführungsform weist das Polypeptid, "Gelatine" eine Bloom-Zahl im Bereich von 0 bis 200 bevorzugt von 40 bis 120 auf und noch bevorzugter um die 100 auf.

In einer weiteren bevorzugten Ausführungsform hat das Polyethylenglykol ein Molekulargewicht (Gewichtsmittel) von mindestens 200 g/mol bis 35000 g/mol bevorzugt in einem Bereich von 400 g/mol bis 20000 g/mol und noch bevorzugter im Bereich von 800 g/mol bis 2000 g/mol.

Zur Erzeugung eines Films wird die Zusammensetzung vorzugsweise im Solzustand auf einen Untergrund aufgetragen bzw. gegossen, und anschliessend vorzugsweise durch Abkühlen des Untergrundes oder aber durch Erkalten lassen der Zusammensetzung an der Umgebungstemperatur in ein Gel überführt. Die Filme können bei entsprechendem Wassergehalt, d.h. vor Trocknung, sehr elastisch sein (Wassergehalt von 10% bis 50 Gew.% bezogen auf das Gesamtgewicht der Zusammensetzung). Die Filme können je nach Wahl des Weichmachers und Weichmacheranteils hohe Bruchdehnungen aufweisen. Hohe Bruchdehnungswerte vor allem bei Verarbeitungstemperatur erlauben starke Verformungen der Filme, bzw. der daraus hergestellten Formkörper. So können die Filme in einem der bekannten Verkapselungsprozesse zu Kapselhüllen geformt werden. Die Kapsel bzw. der Formkörper wird nach der Herstellung einem Trocknungsprozess unterworfen. Der Wassergehalt der Kapselhülle beträgt nach Trocknung ca. 10 Gew. % bezogen auf das Gesamtgewicht der Kapselhülle.

Zur Erzeugung des Gels wird in einer bevorzugten Ausführungform das amingruppenhaltige Polymer, insbesondere das Polypeptid "Gelatine" mit Polyolen, vorzugsweise mit Polyethylenglykol, "titriert". Dabei wird das Polyol dem depolymerisierten Polymer bis zum Erreichen einer vorgegebenen Endviskosität der Zusammensetzung langsam unter gleichmässigem Rühren zugegeben. Der Übergang zum Gel erfolgt sichtbar schlagartig. Alternativ kann das Polyethylenglykol jedoch auch bei der Depolymerisation der amingruppenhaltigen Polymeren, insbesondere "Gelatine" anwesend sein. Die Bedingungen der Depolymerisation sollten dabei jedoch so gewählt werden, dass das Polyethylenglykol nicht wesentlich angegriffen wird, d.h. der Polymerisationsgrad des Polyethylenglykols sollte sich nicht wesentlich ändern.

Bei Überführung der "Gelatine" in ein Gel mittels Polyethylenglykol erfolgt der Übergang vom flüssigen in den gelförmigen Zustand so unzweideutig und schlagartig, dass diese Methode zur titrimetrischen Bestimmung der Bloom-Zahl der vorgelegten "Gelatine" herangezogen werden kann.

Die Zusammensetzung kann zusätzliche Substanzen, wie Farbstoffe, Füllstoffe, Pigmente, Konservierungsmittel, Geschmacksstoffe, zusätzliche Polymere, Weichmacher und Lösungsmittel enthalten. Insbesondere durch Beimischung geeigneter Weichmacher, wie z.B. Glycerin, Sorbitol, Ethylenglykol, Propylenglykol, Polypropylenglykole und Polyglycerine kann der Gelpunkt des Gels, sowie die Viskosität, Bruchdehnung und Elastizität des Gelmaterials geeignet eingestellt werden.

Durch das flexible Einstellen des Gelpunktes der Zusammensetzung kann die Lösetemperatur der aus der erfindungsgemässen Zusammensetzung hergestellten Formkörper reguliert und eingestellt werden.

Eine bevorzugte Ausführungsform der vorliegenden Erfindung umfasst eine Kapselhülle, die die erfindungsgemäss gelbildende Zusammensetzung umfasst. Die Kapselhülle kann in Stanz-, Spritz-, Tauch-, Tropf- oder Blasverfahren hergestellt werden. Einen besonderen Stellenwert besitzt die Herstellung von Kapseln mit einteiliger Kapselhülle, die in intermittierenden oder kontinuierlichen Formverfahren bzw. Form- und Füllverfahren hergestellt werden.

Besonders bevorzugt ist dabei das Rotary-Die-Verfahren. (Die Kapsel-Grundlagen, Technologie und Biopharmazie einer modernen Arzneiform, Hrsg. von Wolfgang Fahrig und Ulrich Hofer, Wissenschaftliche Verlagsgesellschaft mbH, Stuttgart 1983) Die Gelbänder, die mittels des Rotary-Die-Verfahrens zu Weichkapseln geformt werden, müssen Bruchdehnungen von mindestens 100 % aufweisen. Mit einteiligen Kapselhüllen werden in der vorliegenden Anmeldung Kapselhüllen bezeichnet, die ohne Zerstörung bzw. Beschädigung der Kapselhülle nicht wieder getrennt werden können. Für Gelatine werden diese Kapseln im Stand der Technik als Weichgelatinekapseln bezeichnet.

Hart(gelatine)kapseln bestehen dagegen aus zwei Teilen, die zusammengesteckt werden. Sie eignen sich im Gegensatz zu Weichkapseln nicht für das Verkapseln flüssiger Substanzen. Die Abgrenzung zwischen Weich- und Hart(gelatine)kapseln soll aufgrund der äusseren Charakteristik der Kapselhülle und nicht aufgrund bestimmter Weichmacheranteile des Hüllmaterials erfolgen, da auch Hart(gelatine)kapseln mit relativ hohem Weichmacheranteil bekannt sind. In der Regel werden sie durch Spritzgiessen hergestellt. Die Zusammensetzung der vorliegenden Erfindung eignet sich bei Wassergehalten bis 10 Gew. % bezogen auf das Gesamtgewicht, vorzugsweise 6 bis 8 Gew. % auch für das Spritzgussverfahren.

Ist die erfindungsgemässe Zusammensetzung so eingestellt, dass sie einen hohen Gelpunkt besitzt, so können damit Kapselinhaltsstoffe bei Temperaturen verkapselt werden, die mit konventionellen Weichkapselhüllen, insbesondere wenn diese aus Gelatine hergestellt sind, ein Auflösen der Kapselhülle mit sich bringt.

Es können auch wässrige Inhaltsstoffe verkapselt werden, solange die Temperatur des wässrigen Inhaltsstoffes den Gelpunkt der Kapselhülle nicht übersteigt.

Teil der vorliegenden Erfindung ist weiterhin ein Verfahren zum Herstellen eines kaltwasserlöslichen filmbildenden Gelatinegels, wobei das Verfahren die folgenden Schritte umfasst:
a) Erzeugen eines Hydrolysats aus Gelatine, mit einer Bloom-Zahl zwischen 40 und 120, vorzugsweise 100.
b) Zugabe von Polyethylenglykol mit einem mittleren Molekulargewicht von 200 bis 35000 g/mol (Gewichtsmittel) zu dem unter a) erzeugten Hydrolysats bis ein vorbestimmter Viskositätswert bei einer vorbestimmten Temperatur erreicht ist.

In einer bevorzugten Ausführungsform hat das Polyehtylenglykol ein mittleres Molekulargewicht im Bereich von 400 g/mol bis 20000 g/mol und noch bevorzugter um die 800 g/mol bis 2000 g/mol.

In einer bevorzugten Ausführungsform ist das Hydrolysat erzeugt aus einer Gelatine mit einer Bloom-Zahl von mindestens 150 und insbesondere einem Wassergehalt von 4 bis 7 Gew.% bezogen auf das Gesamtgewicht der Gelatine.

Vorzugsweise wird die Depolymerisation im erfindungsgemässen Verfahren unter thermischen Bedingungen ausgeführt. Die Depolymerisation der Gelatine wird unter Konstanthalten der Temperatur auf 90°C bis 110°C, vorzugsweise 100°C in einem Zeitraum von 6 bis 8 Tagen, vorzugsweise 7 Tage durchgeführt. Die Höhe der Temperatur und Dauer des Vorgangs richtet sich a.) nach dem gewünschten Polymerisationsgrad bzw. Bloom-Zahl und b) nach dem Polymerisationsgrad bzw. Bloom-Zahl des Ausgangsmaterials. Das Polyethylenglykol kann in einem alternativen Verfahren wenigstens teilweise bereits schon vor bzw. während der Depolymerisation der Gelatine vorhanden sein. Die Feineinstellung der Endviskosität kann dann, falls erforderlich, nach Abschluss der Depolymerisation durchgeführt werden.

## Patentansprüche

1. Verwendung mindestens eines Polyols als Gelbildner für flüssige Mischungen enthaltend mindestes ein aminogruppenhaltiges Polymer.

2. Polyol für die Verwendung nach Anspruch 1, **dadurch gekennzeichnet, dass** das Polyol Polyethylenglykol umfasst mit einem mittleren Molekulargewicht im Bereich von 200 bis 35000 g/mol (Gewichtsmittel) bevorzugt von 400 bis 20000 g/mol und noch bevorzugter von 800 bis 2000 g/mol.

3. Aminogruppenhaltiges Polymer für die Verwendung nach Anspruch 1 oder 2, **dadurch gekennzeichnet, dass** das Polymer ausgewählt ist aus der Gruppe der Polypeptide.

4. Polypeptide für die Verwendung nach Anspruch 1 bis 3, **dadurch gekennzeichnet, dass** das Polypeptid ausgewählt ist aus der Gruppe der nativen Gelatine und Gelatinehydrolysate.

5. Gelatinehydrolysate für die Verwendung nach Anspruch 1 bis 4, **dadurch gekennzeichnet, dass** das Gelatinehydrolysat eine Bloom-Zahl von 40 bis 120, vorzugsweise 100 aufweist.

6. Verwendung eines Polyols für Mischungen entsprechend einem der Ansprüche 1 bis 5, **dadurch gekennzeichnet, dass** die Mischung 2 bis 80 Gew.% Wasser bezogen auf ihr Gesamtgewicht der Mischung enthält.

7. Zusammensetzung für die Herstellung von Filmen und Formkörper, enthaltend mindestens ein Polyol, insbesondere ein Polyethylenglykol mit einem Molekulargewicht im Bereich von 200 bis 35000 g/mol, vorzugsweise von 400 bis 20000 g/mol, bevorzugt von 800 bis 2000 g/mol (Gewichtsmittel)und Gelatinehydrolysat mit einer Bloom-Zahl im Bereich von 40 bis 120, vorzugsweise 100.

8. Zusammensetzung für die Herstellung von Filmen und Formkörpern, enthaltend mindestens ein Polyol, insbesondere ein Polyethylenglykol mit einem Molekulargewicht im Bereich von 200 bis 35000 g/mol, vorzugsweise von 400 bis 20000 g/mol, bevorzugt von 800 bis 2000 g/mol (Gewichtsmittel) und eine Gelatine mit einer Bloom-Zahl von grösser oder gleich 170, bevorzugt grösser oder gleich 200.

9. Kapsel, bestehend aus einteiliger Kapselhülle und Kapselinhalt, **dadurch gekennzeichnet, dass** die Kapselhülle eine Zusammensetzung nach Anspruch 7 oder 8 enthält.

10. Verfahren zum Herstellen eines kaltwasserlöslichen Gelatinegels, umfassend die Schritte
a) Bereitstellen eines Hydrolysats aus Gelatine, vorzugsweise mit einer Bloom-Zahl zwischen 40 und 120, vorzugsweise 100;
b) Zugabe von Polyethylenglykol mit einem Molekulargewicht von 200 bis 35000 g/mol (Gewichtsmittel) vorzugsweise 400 bis 20000 g/mol und noch bevorzugter 800 bis 2000 g/mol zu dem unter a) bereitgestellten Hydrolysats bis ein vorbestimmter Viskositätswert bei einer vorbestimmten Temperatur erreicht ist.

11. Verfahren zum Herstellen einer Kapsel nach Anspruch 9 durch das Rotary-Die-Verfahren.
